# EUROPÄISCHE PATENTSCHRIFT

(11) **EP 0 333 990 B1**
(45) Veröffentlichungstag und Bekanntmachung des Hinweises auf die Patenterteilung: **21.07.1993**
(21) Anmeldenummer: 89101147.0
(22) Anmeldetag: 23.01.1989
(51) Int. Cl.: A61F 2/46, A61F 2/44, A61B 17/28

(54) **Chirurgischer Instrumentensatz**
Surgical instrument set
Jeu d'instruments chirurgical

(30) Priorität: 23.03.1988 DE 3809793
(43) Veröffentlichungstag der Anmeldung: 27.09.1989
(73) Patentinhaber: Waldemar Link (GmbH & Co.), D-22315 Hamburg (DE)
(72) Erfinder: Keller, Arnold, D-2061 Kayhude (DE)
(74) Vertreter: Glawe, Delfs, Moll & Partner

(56) Entgegenhaltungen:
- EP-A- 0 176 728
- EP-A- 0 269 935
- DE-A- 3 023 942
- US-A- 3 486 505
- ZEITSCHRIFT FUER ORTHOPAEDIE UND IHRE GRENZGEBIETE Band 125, 1987, Seiten 1-6, Stuttgart, D; K.BUETTNER-JANZ et al.: "Eine alternative Behandlungsstrategie beim lumbalen Bandscheibenschaden mit der Bandscheibenendoprothese Modulartyp SB Charité" * Seite 2, rechte Spalte, Zeilen 1-15 *

## Beschreibung

Die Erfindung betrifft einen chirurgischen Instrumentensatz zum Einsetzen von Zwischenwirbel-Endoprothesen und Zwischenwirbel-Endoprothese, die aus zwei Abschlußplatten und einem dazwischen anzuordnenden Gleitkern besteht, wobei der Instrumentensatz eine Spreizzange aufweist.

Es ist bekannt, in der Wirbelsäule krankhaft veränderte oder nicht mehr funktionsfähige Bandscheiben durch Zwischenwirbel-Endoprothesen zu ersetzen (Artikel von Büttner-Janz, K., Schellnack, K., Zippel, H. "Eine alternative Behandlungsstrategie beim lumbalen Bandscheibenschaden mit der Bandscheibenendoprothese Modulartyp SB Charité", Z. Orthop. 125 (1987), 1-6). Diese Zwischenwirbel-Endoprothesen werden anstelle der vorher entfernten Bandscheibe zwischen die Wirbel eingesetzt. Die beiden Abschlußplatten weisen dabei zackenförmige Vorsprünge auf, durch die sie an den Wirbelkörpern festgehalten werden. Auf ihren zueinander zugewendeten Flächen weisen sie kugelschalenförmige Ausnehmungen auf, zwischen die ein Gleitkern gesetzt wird, der ähnlich geformte kugelschalenförmige Vorsprünge hat. Auf diese Weise ist nach der Operation wieder eine Relativbewegung der Wirbel zueinander möglich. Die Abschlußplatten der Endoprothese bestehen dabei aus Metall, während der Gleitkern aus Kunststoff besteht.

Ein großes Problem besteht beim Einsetzen dieser Zwischenwirbel-Endoprothesen. Um die Zwischenwirbel-Endoprothese einsetzen zu können, müssen die sich gegenüberstehenden Wirbelkörper auseinandergespreizt werden. Der Einsatz von bekannten Spreizzangen, die üblicherweise für Operationen an der Bandscheibe benutzt werden, würden aber den Platz besetzen, den die Zwischenwirbel-Endoprothese einnehmen soll. Dabei ist zu beachten, daß die Zwischenwirbel-Endoprothese, zur günstigen Lastverteilung, möglichst großflächig, dem Maß der Wirbelkörper entsprechend, aufliegen soll. Ebenfalls erfordert das Auseinanderspreizen der sich gegenüberstehenden Wirbelkörper beträchtliche Kräfte.

Mit den bisher üblichen Spreizzangen könnte somit allenfalls nur an den äußeren Rändern der Wirbelkörper angegriffen werden, die nicht von der Zwischenwirbel-Endoprothese besetzt sind. Diese, nur bedingt vorhandenen Knochenflächen der Wirbelkörper, wären aber zum Aufbringen der notwendigen Kräfte zu klein und provozieren damit die Gefahr, daß die Wirbelkörper an diesen Stellen einbrechen.

Die Aufgabe der Erfindung besteht in der Schaffung eines Instrumentensatzes, mit dem die Endoprothesen bei gleichzeitigem Spreizen der Wirbelkörper und möglichst geringem Risiko des Einbrechens der Wirbelkörper eingesetzt werden können.

Die erfindungsgemäße Lösung besteht darin, daß die Spreizzange an ihrem vorderen Ende an jeder Spreizbacke eine eine Abschlußplatte an drei Seiten umgreifende und am Rand festhaltende im wesentlichen U-förmige Ausnehmung aufweist, wobei die Dicke der Spreizbacken in Spreizrichtung im wesentlichen gleich der Dicke der Abschlußplatten ist.

Die Abschlußplatten werden also auf drei Seiten von den Spreizbacken umgriffen. Die beiden Abschlußplatten liegen dabei zunächst direkt aufeinander. In dieser Stellung werden dann die Spreizbacken zwischen die beiden Wirbelkörper gebracht. Anschließend erfolgt dann das Spreizen, damit Raum für den Gleitkern geschaffen wird, wobei das Spreizen noch in größerem Ausmaß erfolgen muß, da zunächst der Gleitkern mit seinen Vorsprüngen zwischen die Abschlußplatten eingebracht werden muß.

Die dabei auftretenden beträchtlichen Kräfte werden nicht nur auf kleine Bereiche der Wirbelkörper übertragen, sondern großflächig. Diese Kraft wird dabei durch die Abschlußplatten ausgeübt. Auf diese Weise ist mit größtmöglicher Sicherheit das Einbrechen der Wirbelkörper vermieden. Die Wirbelkörper kommen im wesentlichen nur mit den Teilen in Berührung, mit denen sie auch nach der Operation in Berührung stehen, nämlich den Abschlußplatten.

Zweckmäßigerweise sind die Spreizbacken im Bereich der Innenseiten der Schenkel des U mit Nuten zum Aufnehmen der Abschlußplatten versehen, die in der zur Spreizrichtung senkrechten Ebene angeordnet sind. Dadurch können die Abschlußplatten vom offenen Ende des U seitlich in die Spreizbacken eingeschoben werden. Die großen Spreizkräfte, die auf die Abschlußplatten wirken, werden dabei von den Seitenwänden der Nuten aufgenommen. Die Kraft wirkt dabei nur in einer Richtung, so daß nur eine Nutenwand besonders kräftig ausgebildet sein muß. Durch die äußere Nutenwand muß lediglich das Herausfallen der Abschlußplatten verhindert werden. Hier können die Nutenwände schwächer ausgebildet sein, wobei vorgesehen sein kann, daß die Nuten abgeschrägte Wände aufweisen, da dann auch die Abschlußplatten an den entsprechenden Stellen entsprechend abgeschrägte Wände besitzen können, so daß hier keine scharfen Kanten vorhanden sind.

Damit die Abschlußplatten nicht nach vorne aus der Öffnung des U herausrutschen können, kann bei jeder Spreizbacke an einem vorderen Nutenende eine federnd nachgebende Klinke vorgesehen sein. Ist eine Abschlußplatte in die Spreizbacke eingeschoben, so hindert die federnd nachgebende Klinke dieselbe daran, herauszufallen. Ist andererseits die Endoprothese eingesetzt, so wird die Klinke nachgeben, wenn man die Zange wieder entfernen will.

Zweckmäßigerweise wird man die Abschlußplatten beim Spreizvorgang so auseinanderbewegen, daß ihre an den Wirbelkörpern angreifende Oberflächen parallel bleiben. Daher wird zweckmäßigerweise vorgesehen, daß die Spreizbacken mit einer scherenartigen Parallelführung verbunden sind.

Zweckmäßigerweise wird man vorsehen, daß die Spreizzange eine lösbare Rasteinrichtung für die gespreizte Stellung aufweist, damit der Operateur nach dem Spreizen die Hände frei hat, um den Gleitkern einzusetzen.

Das Einschieben der Spreizzange mit den daran angeordneten Abschlußplatten zwischen die Wirbelkörper erfordert verhältnismäßig viel Kraft, die allein durch die Spreizzange sehr schwer oder gar nicht übertragen werden kann. Deswegen ist zweckmäßigerweise vorgesehen, daß die Spreizzange im Bereich der Spreizbacken mit einem Vorsprung oder einer Ausnehmung versehen ist, der bzw. die eine im wesentlichen quer zur Eintreibrichtung ausgerichtete Anschlagf läche für ein im wesentlichen stabförmiges Eintreibinstrument aufweist. Die Kraft zum Einschieben der Spreizzange wird also mit Hilfe eines Eintreibinstruments aufgebracht, mit dem die Eintreibkraft in der richtigen Richtung ausgeübt werden kann, was insbesondere bei abgewinkelten Spreizbacken nicht möglich ist. Die Anschlagfläche kann dabei zylindrischen oder kugelförmigen Querschnitt haben, so daß das Eintreibinstrument unter verschiedenen Winkeln angesetzt werden kann.

Wie bereits erwähnt sind die Spreizkräfte sehr groß. Da die Spreizzange verhältnismäßig lange Hebelarme hat, würde sie sehr unförmig werden, wenn man sie so stabil ausbildet, daß sie in jedem Fall die notwendigen Spreizkräfte ausüben kann. Zweckmäßigerweise ist daher vorgesehen, daß der Instrumentensatz ein im wesentlichen schraubendreherähnliches stabförmiges Element aufweist, das an seinem einen Ende einen Handgriff trägt, wobei das andere Ende die Form einer länglichen Platte hat. Nachdem die Spreizung begonnen worden ist und die Spreizbacken schon einen gewissen Abstand voneinander haben, kann das stabförmige Element zwischen die Backen so eingeführt werden, daß die Ebene der länglichen Platte im wesentlichen zur Ebene der Spreizbacken parallel ist. Wird dann anschließend das stabförmige Element gedreht, so wird genau an der Stelle, an der die Spreizkraft erforderlich wird, eine sehr große Kraft ausgeübt, bis die Ebene der Platte im wesentlichen senkrecht zur Spreizbackenebene steht. In dieser Stellung kann dann das stabförmige Element in der Spreizzange verbleiben, um diese Stellung zu fixieren. Gegebenenfalls kann diese Spreizung schrittweise dadurch erweitert werden, daß man anschließend ein stabförmiges Element mit einer etwas breiteren Platte verwendet, um so allmählich zu größeren Breiten zu kommen.

Statt einer Platte mit im wesentlichen rechteckigem Querschnitt und abgerundeten Kanten könnte man auch z.B. ein ovales Teil verwenden.

Besonders zweckmäßig können die Gleitkerne eingesetzt werden, wenn der Instrumentensatz ein Halteinstrument für Gleitkerne aufweist, das stabförmig ist und am vorderen Ende mit federnden, dem Gleitkern am Umfang über einen Winkelbereich von etwas mehr als 180° umgreifenden Halteelementen versehen ist.

Die federnden Halteelemente halten zunächst den Gleitkern fest. Ist die Spreizung aufgehoben und wird der Gleitkern zwischen den Abschlußplatten festgehalten, so kann das Halteinstrument wieder herausgezogen werden, wobei sich die Halteelemente aufgrund ihrer Federwirkung vom Gleitkern lösen, der am gewünschten Ort verbleibt.

Zweckmäßigerweise sind zwei einstückig miteinander verbundene Halteelemente vorgesehen, die sich über je einen Winkelbereich von ungefähr 90° erstrecken, die mit einer Stange verbunden sind, die sich durch das rohrförmig ausgebildete Halteinstrument erstreckt, wobei die Halteelemente mit nach vorne auseinanderlaufenden Schrägflächen innen am Rohrende anliegen, und wobei am anderen Ende eine Schraubenanordnung zum Ausüben einer Zugkraft auf die Stange vorgesehen ist. Aufgrund der Federwirkung werden bei dieser Ausführungsform die beiden Halteelemente auseinandergedrückt, so daß sich eine Öffnung bildet, in die der Gleitkern eingeführt werden kann. Wird anschließend eine Zugkraft auf die Stange ausgeübt, so berühren die Schrägflächen innen das Rohr, so daß die Halteelemente gegen die Federkraft aufeinander zugedrückt werden und somit den Gleitkern festhalten. Damit der Gleitkern anschließend aus dem Halteinstrument entfernt werden kann bzw. das Halteinstrument vom Gleitkern abgezogen werden kann, muß dann durch Drehen der Schraubanordnung in der entgegengesetzen Richtung lediglich die Zugkraft aufgehoben werden.

Das Halteinstrument ist besonders zweckmäßig im Zusammenhang mit der Spreizzange zu verwenden, kann aber auch für andere Arten des Einsetzens der Endoprothesen verwendet werden. Zur Erfindung gehört daher auch ein Instrumentensatz, der nur ein oder mehrere Halteinstrumente aufweist.

Wenn der Instrumentensatz weiterhin ein stangenförmiges Element aufweist, an dem vorne ein Modell eines Gleitkerns befestigt ist, so kann mit diesen Elementen nach Durchführung der Spreizung festgestellt werden, welcher Gleitkern am besten paßt. Zu diesem Zweck wird man selbstverständlich mehrere solcher stangenförmiger Elemente mit unterschiedlichen Gleitkernen verwenden. Man probiert also die richtige Größe mit Modellen von Gleitkernen aus und nicht mit Gleitkernen, von denen dann anschließend einer in der Endoprothese verbleiben soll. Dies ist unter anderem schon aus Gründen der Sterilisierung zweckmäßiger, da ein dauernd im Körper verbleibender Gleitkern sorgfältiger sterilisiert werden muß als ein Modell eines Gleitkerns, der nach kurzer Zeit wieder entfernt wird.

Zweckmäßigerweise wird der Instrumentensatz nicht nur mehrere stangenförmige Elemente mit Modellen von Gleitkernen, sondern auch mehrere Spreizzangen, schraubendreherähnliche Spreizelemente und Halteinstrumente für Gleitkerne aufweisen, damit verschieden große Endoprothesen eingesetzt werden können.

Die Erfindung wird im folgenden anhand von vorteilhaften Ausführungsformen unter Bezugnahme auf die beigefügten Zeichnungen beschrieben. Es zeigen:
- Fig. 1: im Querschnitt eine Zwischenwirbel-Endoprothese, die mit dem erfindungsgemäßen Instrumentensatz eingesetzt werden kann;
- Fig. 2: eine Spreizzange der Erfindung in Seitenansicht;
- Fig. 3: ein Detail der Spreizzange der Fig. 2 in Draufsicht;
- Fig. 4: eine vergrößerte Darstellung des Teiles der Fig. 3;
- Fig. 5: eine Querschnittsansicht entlang der Linie V-V von Fig. 4;
- Fig. 6: eine Querschnittsansicht entlang der Linie VI-VI von Fig. 4;
- Fig. 7: eine Gesamtansicht eines Halteinstruments für Gleitkerne;
- Fig. 8 und 9: Querschnittsansichten des Instruments der Fig. 7 in verschiedenen arbeitsmäßigen Stellungen;
- Fig. 10: ein Eintreibinstrument, das mit der Spreizzange der Fig. 2 bis 6 verwendet werden kann;
- Fig. 11: ein zusätzliches schraubendreherförmiges Spreizinstrument;
- Fig. 12: ein stangenförmiges Element mit einem Modell eines Gleitkerns.

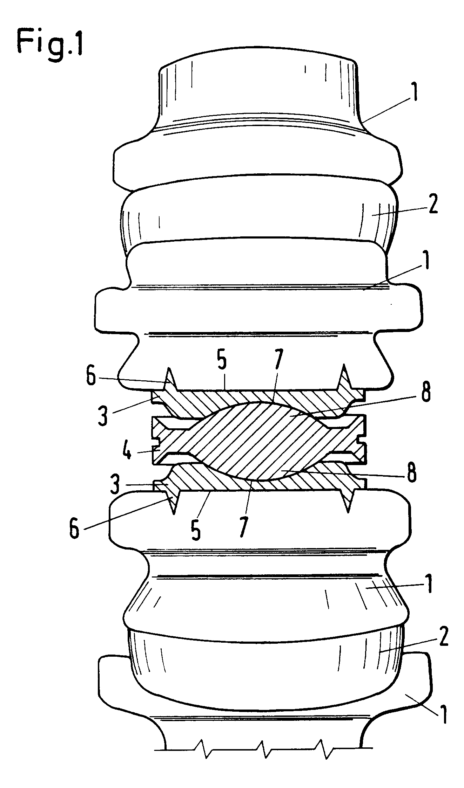

In Fig. 1 ist im Querschnitt ein Teil einer Wirbelsäule mit darin eingesetzter Endoprothese dargestellt. Die Wirbelsäule besteht aus einer Vielzahl von Wirbelkörpern 1, zwischen denen beim gesunden Menschen Bandscheiben 2 angeordnet sind. Die Bandscheibe zwischen den beiden mittleren Wirbelkörpern 1 ist durch die Zwischenwirbel-Endoprothese ersetzt, die im Zusammenhang mit dem erfindungsgemäßen Instrumentensatz verwendet werden kann. Die Endoprothese besteht aus zwei Abschlußplatten 3 und einem zwischen denselben angeordneten Gleitkörper 4. Auf der zum Wirbelkörper 1 gerichteten Seite weist die Abschlußplatte 3 eine im wesentlichen ebene Oberfläche 5 auf, die mit zackenartigen Vorsprüngen 6 versehen ist, die in den Wirbelkörper eindringen, um so die Abschlußplatten 3 sicher am Wirbelkörper 1 festzuhalten. Auf der gegenüberliegenden Seite sind die Abschlußplatten 3 mit einer im wesentlichen kugelschalenförmigen Ausnehmung 7 versehen. Die Abschlußplatten 3 bestehen normalerweise aus Metall.

Zwischen den Abschlußplatten 3 ist der Gleitkern 4 angeordnet, der zu den kugelschalenförmigen Ausnehmungen 7 entsprechende kugelschalenförmige Vorsprünge 8 aufweist. Der Gleitkörper 4 besteht dabei normalerweise aus Kunststoff.

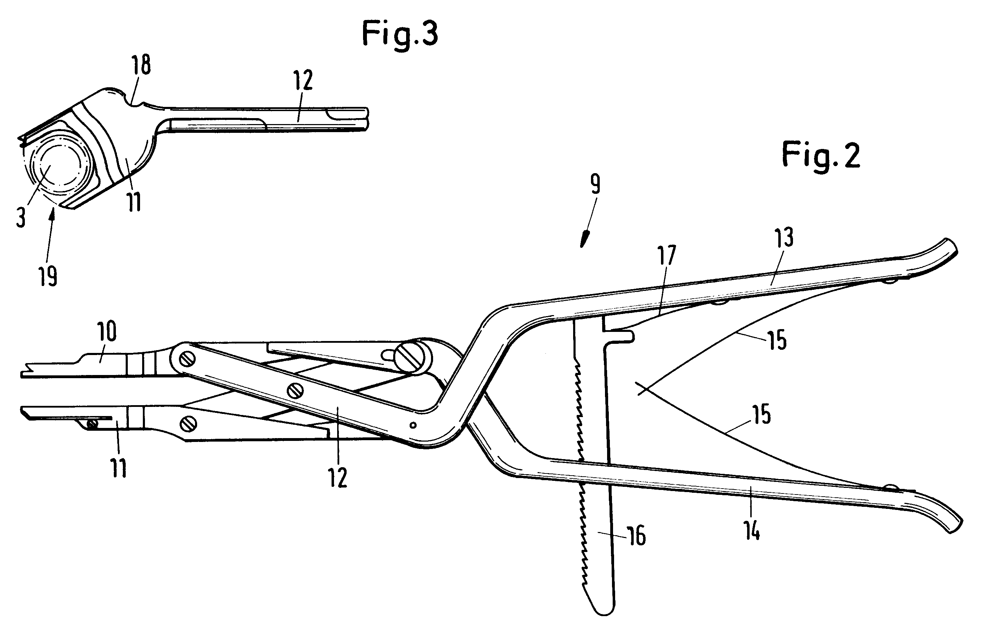

Die in den Fig. 2 bis 6 gezeigte Spreizzange 9 weist zwei parallel angeordnete Spreizbacken 10, 11 auf, die durch ein scherenartiges Gelenk 12 mit Hilfe von Hebeln 13, 14 so auseinander gespreizt werden können, daß ihre Flächen parallel bleiben. Die Hebel 13, 14 werden dabei durch Federn 15 auseinandergedrückt, so daß die Spreizbacken 10, 11 normalerweise zusammengedrückt sind. Werden gegen die Kraft der Feder 15 die Hebel 13, 14 zusammengedrückt, so werden die Spreizbacken 10, 11 auseinandergedrückt. Dabei rasten Zähne eines Sperrelementes 16, das durch eine Feder 17 in Fig. 2 im Uhrzeigersinn federbelastet ist, in entsprechende Zähne oder Vorsprünge des unteren Hebels 14 ein, so daß die jeweils erreichte Spreizstellung durch das Element 16, das schwenkbar am Hebel 13 befestigt ist, gehalten wird.

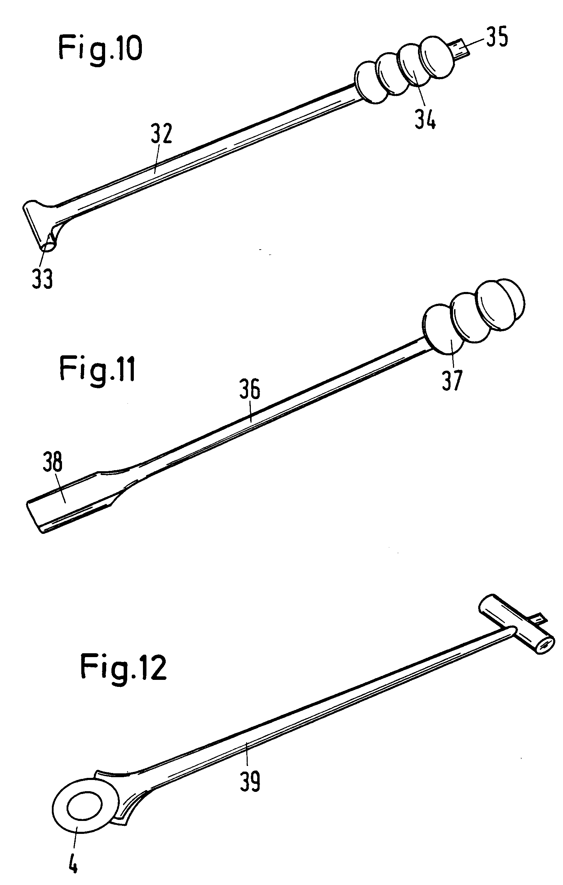

Wie dies in Fig. 3 ersichtlich ist, sind die Spreizbacken 10, 11 relativ zur Zangenlängsrichtung abgewinkelt, da man so die Spreizbacken besser in den freigelegten Raum zwischen die Wirbelkörper führen kann. Da hierzu normalerweise große Kräfte nötig sind, weisen die Spreizbacken 10, 11 an ihrem hinteren Ende eine zylinderförmige Ausnehmung 18 auf, an der ein Eintreibinstrument angesetzt werden kann, das in Fig. 10 gezeigt ist.

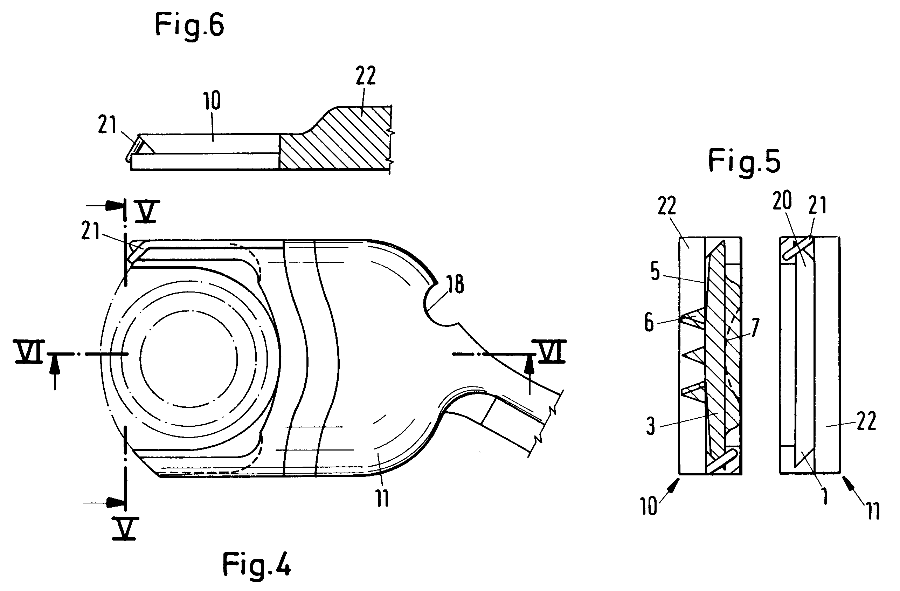

Die Spreizbacken 10, 11 weisen an ihrem vorderen Ende eine U-förmige Ausnehmung 19 auf, in die eine Abschlußplatte 3 eingesetzt werden kann. In der Querschnittsansicht der Fig. 5 ist dabei nur eine dieser Abschlußplatten 3 dargestellt. Die Abschlußplatten 3 werden in Nuten 20 gehalten, die sich entlang der Schenkel des U erstrecken. Die Abschlußplatten 3 werden dabei lediglich am Rand in entsprechenden Nuten 20 der Spreizbacken 10, 11 festgehalten, die auf einer Seite abgeschrägt sind. Auf der Seite, auf der durch die Abschlußplatten 3 die größte Kraft ausgeübt ist, ist der Nutgrund aber eben und im wesentlichen parallel zur Spreizbackenebene.

In den Fig. 4 bis 6 erkennt man noch eine federnde Klinke 21, die den Nutausgang normalerweise verschließt und dadurch eine dort eingeschlossene Abschlußplatte 3 festhält. Diese federnde Klinke 21 gibt aber nach, wenn gewollt eine Abschlußplatte in die Nut 20 hineingesteckt werden soll oder die Spreizzange nach Einbringen der Endoprothese abgezogen werden soll.

Wie man aus den Fig. 4 bis 6 erkennt, haben die Spreizbacken 10, 11 im vorderen Teil, der zwischen die Wirbelkörper verbracht wird, im wesentlichen die Dicke der Abschlußplatten 3. Die Zähne 6 ragen dabei über diesen Bereich heraus. Erst hinter dem Bereich der Spreizbacken, der zwischen die Wirbelkörper eingebracht wird, haben die Spreizbacken eine die Stabilität erhöhende Verdickung 22.

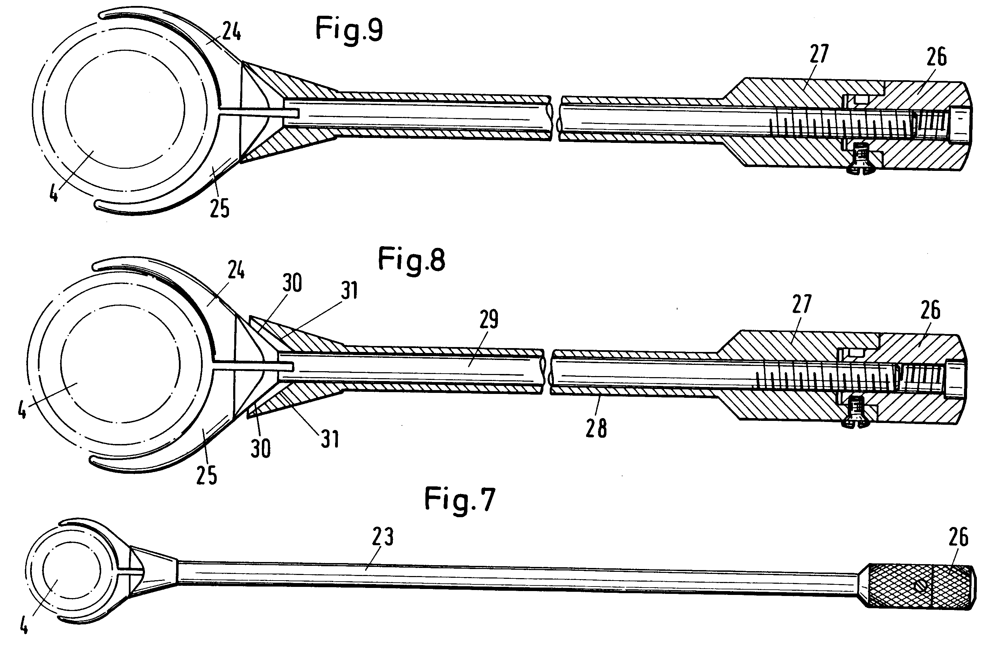

In den Fig. 7 bis 9 ist ein Halteinstrument 23 für Gleitkerne 4 dargestellt. Fig. 7 ist dabei eine Ansicht. Fig. 8 und 9 sind Querschnittsdarstellungen in zwei verschiedenen Funktionsstellungen.

Das Halteinstrument 23 weist ein im wesentlichen U-förmiges Halteelement auf, das aus zwei Schenkeln 24, 25 besteht. Die Schenkel nehmen dabei normalerweise die in Fig. 8 gezeigte Ruhelage ein, bei der die Schenkel 24, 25 den Gleitkern 4 nur verhältnismäßig lose umfassen. Wird aber an der Schraube 26 am hinteren Ende eines Handgriffes 27 gedreht, so wird auf eine in einem Außenrohr 28 angeordnete Zugstange 29, die mit den Halteelementteilen 24, 25 verbunden ist, eine Zugkraft ausgeübt. Dadurch stoßen die Halteelementteile 24, 25 mit abgeschrägten Flächen 30 gegen entsprechende abgeschrägte Flächen 31 des Rohres 28 bzw. eines Ansatzes an demselben, so daß die beiden Teile 24, 25 zusammengedrückt werden und den Gleitkern 4 festhalten.

In Fig. 10 ist ein Eintreibinstrument 32 gezeigt, das im wesentlichen stabförmig ausgebildet ist. An seinem vorderen Ende trägt es ein querstehendes zylindrisches Teil 33, das in die Ausnehmung 18 der Spreizzange 9 der Fig. 2 bis 4 eingesetzt werden kann. Hält man dann das Eintreibinstrument 32 am Handgriff 34 fest und übt eine Schlagkraft auf das dem zylindrischen Teil 33 entgegengesetzte Ende 35 auf, so kann die Spreizzange 9 mit den daran angeordneten Abschlußplatten in den Raum zwischen den Wirbelkörpern eingebracht werden.

Nachdem die Spreizung bis zu einem gewissen Ausmaß vorgenommen worden ist, kann die weitere Spreizung mit Hilfe des Instrumentes 36 der Fig. 11 erfolgen. Dieses Instrument 36 besitzt ebenfalls einen Handgriff 37 und ist am anderen Ende schraubendreherartig zu einer Platte 38 verbreitert. Diese Platte 38 wird zwischen die Spreizbacken in einer Stellung eingebracht, in der die Ebene der Platte 38 im wesentlichen parallel zur Ebene der Spreizbacken angeordnet ist oder zumindest keinen allzu großen Winkel einschließt. Durch Drehen des Instruments 36 können dann die Spreizbacken weiter auseinandergedrückt werden.

In Fig. 12 ist schließlich noch ein stangenförmiges Element 39 gezeigt, das vorne das Modell eines Gleitkerns 4 trägt. Mit Hilfe des Instruments 39 kann durch Probieren herausgestellt werden, welche Gleitkerngröße am besten geeignet ist.

## Patentansprüche

1. Chirurgischer Instrumentensatz zum Einsetzen von Zwischenwirbel-Endoprothesen und Zwischenwirbel-Endoprothese, die aus zwei Abschlußplatten (3) und einem dazwischen anzuordnenden Gleitkern (4) besteht, wobei der Instrumentensatz eine Spreizzange (9) aufweist, dadurch gekennzeichnet, daß die Spreizzange an ihrem vorderen Ende an jeder Spreizbakke (10, 11) eine eine Abschlußplatte (3) an drei Seiten umgreifende und am Rand festhaltende im wesentlichen U-förmige Ausnehmung (19) aufweist, wobei die Dicke der Spreizbacken (10, 11) in Spreizrichtung im wesentlichen gleich der Dicke der Abschlußplatten (3) ist.

2. Chirurgischer Instrumentensatz nach Anspruch 1, dadurch gekennzeichnet, daß die Spreizbacken (10, 11) im Bereich der Innenseiten der Schenkel des U mit Nuten (20) zum Aufnehmen der Abschlußplatten (3) versehen sind, die in der zur Spreizrichtung senkrechten Ebene angeordnet sind.

3. Chirurgischer Instrumentensatz nach Anspruch 2, dadurch gekennzeichnet, daß die Nuten (20) abgeschrägte Wände aufweisen.

4. Chirurgischer Instrumentensatz nach Anspruch 1 oder 2, dadurch gekennzeichnet, daß bei jeder Spreizbacke (10, 11) an einem vorderen Nutenende eine federnd nachgebende Klinke (21) vorgesehen ist.

5. Chirurgischer Instrumentensatz nach einem der Ansprüche 1 bis 4, dadurch gekennzeichnet, daß die Spreizbacken (10, 11) mit einer scherenartigen Parallelführung (12) verbunden sind.

6. Chirurgischer Instrumentensatz nach einem der Ansprüche 1 bis 4, dadurch gekennzeichnet, daß die Spreizzange (9) eine lösbare Rasteinrichtung (16, 17) für die gespreizte Stellung aufweist.

7. Chirurgischer Instrumentensatz nach einem der Ansprüche 1 bis 6, dadurch gekennzeichnet, daß die Spreizzange (9) im Bereich der Spreizbacken (10, 11) mit einem Vorsprung oder einer Ausnehmung (18) versehen ist, der bzw. die eine im wesentlichen quer zur Eintreibrichtung ausgerichtete Anschlagfläche für ein im wesentlichen stabförmiges Eintreibinstrument (32) aufweist.

8. Chirurgischer Instrumentensatz nach einem der Ansprüche 1 bis 7, dadurch gekennzeichnet, daß er ein im wesentlichen schraubendreherähnliches stabförmiges Element (36) aufweist, das an seinem einen Ende einen Handgriff (37) trägt, und daß das andere Ende die Form einer länglichen Platte (38) hat.

9. Chirurgischer Instrumentensatz nach einem der Ansprüche 1 bis 8, dadurch gekennzeichnet, daß er ein Halteinstrument (23) für Gleitkerne (4) aufweist, das stabförmig ist und am vorderen Ende mit federnden, den Gleitkern (4) am Umfang über einen Winkelbereich von etwas mehr als 180° umgreifenden Halteelementen (24, 25) versehen ist.

10. Chirurgischer Instrumentensatz nach Anspruch 9, dadurch gekennzeichnet, daß zwei einstückig miteinander verbundene Halteelemente (24, 25) vorgesehen sind, die sich je über einen Winkelbereich von ungefähr 90° erstrecken, die mit einer Stange (29) verbunden sind, die sich durch das rohrförmig (bei 28) ausgebildete Halteinstrument (23) erstreckt, daß die Halteelemente (24, 25) mit nach vorne auseinanderlaufenden Schrägflächen (30) innen am Rohrende anliegen, und daß am anderen Ende eine Schraubanordnung (26) zum Ausüben einer Zugkraft auf die Stange (29) vorgesehen ist.

11. Chirurgischer Instrumentensatz nach einem der Ansprüche 1 bis 10, dadurch gekennzeichnet, daß er ein stangenförmiges Element (39) aufweist, an dem vorne ein Modell eines Gleitkerns befestigt ist.

12. Chirurgischer Instrumentensatz nach einem der Ansprüche 1 bis 11, dadurch gekennzeichnet, daß er Instrumente (9, 23, 32, 36, 39) verschiedener Größen aufweist.

## Claims

1. Surgical instrument set for the insertion of intervertebral endoprostheses and intervertebral endoprosthesis, consisting of two closing plates (3) and a sliding core (4) to be located between the latter, the instrument set containing expansion forceps (9), characterised in that the expansion forceps have an essentially U-shaped recess (19) at their front end on each expansion jaw (10, 11), which grips a closing plate (3) on three sides and holds it fast on the edge, the thickness of the expansion jaws (10, 11) in the direction of expansion being essentially equal to the thickness of the closing plates (3).

2. Surgical instrument set in accordance with Claim 1, characterised in that the expansion jaws (10, 11) are provided with grooves (20) in the vicinity of the internal sides of the sides of the U, to take up the closing plates (3), these grooves being located in the plane perpendicular to the direction of expansion.

3. Surgical instrument set in accordance with Claim 2, characterised in that the grooves (20) have bevelled sides.

4. Surgical instrument set in accordance with Claims 1 or 2, characterised in that a spring-action catch (21) is provided on the front end of a groove on each expansion jaw (10, 11).

5. Surgical instrument set in accordance with one of Claims 1 to 4, characterised in that the expansion jaws (10, 11) are joined by a scissor-type parallel guide (12).

6. Surgical instrument set in accordance with one of Claims 1 to 4, characterised in that the expansion forceps (9) have a detachable catching device (16, 17) for the expanded position.

7. Surgical instrument set in accordance with one of Claims 1 to 6, characterised in that the expansion forceps (9) are fitted, in the vicinity of the expansion jaws (10, 11), with a projection or recess (18) having a stop face aligned basically transverse to the driving direction for a basically bar-shaped driving instrument (32).

8. Surgical instrument set in accordance with one of Claims 1 to 7, characterised in that it contains a bar-shaped element (36), essentially similar to a screwdriver, with a handle (37) at one end and having the shape of an oblong plate (38) at the other end.

9. Surgical instrument set in accordance with one of Claims 1 to 8, characterised in that it contains a holding instrument (23) for sliding cores (4), which is bar-shaped and is fitted at the front end with sprung holding pieces (24, 25) which grip the circumference of the sliding core (4) over an angle of slightly more than 180°.

10. Surgical instrument set in accordance with Claim 9, characterised in that two single-piece, interconnected holding pieces (24, 25) are provided, each extending over an angle of about 90°, connected by a rod (29), which extends through the holding instrument (23) which is tubular in design (at 28), in that the holding pieces (24, 25) sit inside the end of the tube with slanting surfaces (30) diverging to the front and in that a screw arrangement (26) is provided at the other end to exert traction on the rod (29).

11. Surgical instrument set in accordance with one of Claims 1 to 10, characterised in that it contains a rod-shaped element (39) on the front of which a model of a sliding core is fixed.

12. Surgical instrument set in accordance with one of Claims 1 to 11, characterised in that it contains instruments (9, 23, 32, 36, 39) of different sizes.

## Revendications

1. Jeu d'instruments chirurgicaux pour la mise en place d'endoprothèses intervertébrales, et endoprothèse intervertébrale qui se compose de deux plaques terminales (3) et d'un noyau de glissement (4) à interposer entre ces plaques, le jeu d'instruments comprenant une pince d'écartement (9), caractérisés en ce que la pince d'écartement comporte à son extrémité antérieure, sur chaque mâchoire d'écartement (10, 11), un creux (19) essentiellement en forme d'U qui saisit une plaque terminale (3) sur ses trois côtés et la maintient par le bord, l'épaisseur des mâchoires d'écartement (10, 11) dans la direction d'écartement étant sensiblement égale à l'épaisseur des plaques terminales (3).

2. Jeu d'instruments chirurgicaux selon la revendication 1, caractérisé en ce qu'au niveau des faces internes des branches de l'U, les mâchoires d'écartement (10, 11) sont munies, pour recevoir les plaques terminales (3), de rainures (20) qui sont situées dans le plan perpendiculaire à la direction d'écartement.

3. Jeu d'instruments chirurgicaux selon la revendication 2, caractérisé en ce que les rainures (20) présentent des parois biseautées.

4. Jeu d'instruments chirurgicaux selon la revendication 1 ou 2, caractérisé en ce qu'un cliquet à ressort (21) est prévu sur chaque mâchoire d'écartement (10, 11), à l'extrémité antérieure de la rainure.

5. Jeu d'instruments chirurgicaux selon l'une quelconque des revendications 1 à 4, caractérisé en ce que les mâchoires d'écartement (10, 11) sont reliées à un parallélogramme articulé (12).

6. Jeu d'instruments chirurgicaux selon l'une quelconque des revendications 1 à 4, caractérisé en ce que la pince d'écartement (9) comporte un dispositif d'arrêt par encliquetage déverrouillable (16, 17) pour la bloquer dans la position écartée.

7. Jeu d'instruments chirurgicaux selon l'une quelconque des revendications 1 à 6, caractérisé en ce que la pince d'écartement (9) est munie, dans la région des mâchoires d'écartement (10, 11), d'une saillie ou d'un creux (18) qui présente une surface d'arrêt, essentiellement orientée transversalement par rapport à la direction d'insertion, pour un instrument d'insertion (32) en forme générale de barre.

8. Jeu d'instruments chirurgicaux selon l'une quelconque des revendications 1 à 7, caractérisé en ce qu'il comprend un élément en forme générale de barre (36), semblable à un tournevis, qui porte une poignée (37) à l'une de ses extrémités et dont l'autre extrémité a la forme d'une plaque oblongue (38).

9. Jeu d'instruments chirurgicaux selon l'une quelconque des revendications 1 à 8, caractérisé en ce qu'il comprend un instrument de maintien (23) pour des noyaux de glissement (4), instrument qui est en forme de barre et est muni, à son extrémité antérieure, d'éléments de maintien (24, 25) qui saisissent le noyau de glissement (4) à sa periphérie sur une étendue angulaire d'un peu plus de 180°.

10. Jeu d'instruments chirurgicaux selon la revendication 9, caractérise en ce qu'il est prévu deux éléments de maintien (24, 25) joints d'une seule pièce qui ont chacun une étendue angulaire d'environ 90° et sont raccordés à une tige (29) qui s'étend à travers l'instrument de maintien (23) réalisé sous forme tubulaire (en 28), en ce que les éléments de maintien (24, 25) s'appliquent intérieurement sur l'extrémité du tube par des surfaces obliques (30) qui divergent vers l'avant, et en ce qu'il est prévu, à l'autre extrémité, un dispositif à vis (26) pour exercer une force de traction sur la tige (29).

11. Jeu d'instruments chirurgicaux selon l'une quelconque des revendications 1 à 10, caractérisé en ce qu'il comprend un élément en forme de tige (39) à l'avant duquel est fixé un modèle d'un noyau de glissement.

12. Jeu d'instruments chirurgicaux selon l'une quelconque des revendications 1 à 11, caractérisé en ce qu'il comprend des instruments (9, 23, 32, 36, 39) de tailles différentes.
